# EUROPEAN PATENT SPECIFICATION

(11) **EP 2 877 858 B1**
(45) Date of publication and mention of the grant of the patent: **14.03.2018**
(21) Application number: 13747682.6
(22) Date of filing: 24.07.2013
(51) Int. Cl.: G01N 33/68, C07K 7/08, G01N 33/58

(54) **ISOTOPIC LABELLING OF PROTEINS**
ISOTOPISCHE MARKIERUNG VON PROTEINEN
MARQUAGE ISOTOPIQUE DE PROTÉINES

(30) Priority: 24.07.2012 GB 201213127
(43) Date of publication of application: 03.06.2015
(73) Proprietor: University Of Dundee, Dundee DD1 4HN (GB)
(72) Inventor: HUANG, Jeffrey T-J, Dundee DD1 9SY (GB); WOLF, Roland, Dundee DD1 9SY (GB); MACLEOD, Kenneth, Dundee DD1 9SY (GB)
(74) Representative: HGF Limited
(86) International application number: PCT/GB2013/051966
(87) International publication number: WO 2014/016586

(56) References cited:
- WO-A1-2009/058307
- WO-A1-2011/160045
- ZANIVAN SARA ET AL: "In vivo quantitative proteomics: the SILAC mouse.", METHODS IN MOLECULAR BIOLOGY (CLIFTON, N.J.) 2012, vol. 757, 1 January 2012 (2012-01-01), pages 435-450, XP002713503, ISSN: 1940-6029
- GEIGER TAMAR ET AL: "Use of stable isotope labeling by amino acids in cell culture as a spike-in standard in quantitative proteomics", NATURE PROTOCOLS, NATURE PUBLISHING GROUP, GB, vol. 6, no. 2, 1 February 2011 (2011-02-01), pages 147-157, XP009159759, ISSN: 1750-2799

## Description

The present invention relates to the field of protein quantification and characterisation and, particularly, methods to improve the synthesis of isotope labelled native proteins that are constitutively expressed at low levels in tissues, body fluid, cultured cells, organisms or cell free systems such that metabolic labelled isotopic proteins can be used for protein quantification and characterisation. This invention provides methods to enhance expression of poorly expressed, hard to detect, proteins in a specific, targeted manner.

### Background of the Invention

Mass spectrometry is a powerful technology for qualitative and quantitative analysis. However, quantitative analysis of a molecule (or molecules) using mass spectrometry is not straightforward because the ionization process is affected by environmental conditions (e.g. instrumental parameters) and the chemical composition in each sample. The latter is frequently observed in LC-MS/MS experiments where ion suppression (or in fewer cases, ion enhancement) effect can be highly significant when interfering compounds (or salts) are co-eluted with the analyte(s). The solution to this issue is to introduce an internal standard in mass spectrometry-based analysis such that measurements of analytes are made relative to this internal standard, thus achieving results with a high level of precision and accuracy. To assure a reliable ratio measurement, an internal standard must be a chemical and physical mimic of the analyte. Therefore, isotopically labelled analogues of the analyte are considered to be the best choice since they are biologically and chemically indistinguishable from the analyte. Many gold standard quantification methods for biomarker analysis are based on stable isotope dilution mass spectrometry.

Similar to this concept, isotopically-labelled protein is often used as an internal standard in mass spectrometry-based protein quantification to improve signal inaccuracy caused by variation in sample preparation and by ion suppression/enhancing during ionization. Whilst stable isotopes of small molecules can be synthesised, protein isotopes are more complicated to generate due to the uncertainty of conformation in the synthetic isotopic proteins. Labelling strategies including in vitro labelling and metabolic labelling (where labelling occurs in living organisms or animals) have been developed for this purpose.

One common labelling method uses chemically synthesized isotope-labelled peptides for absolute quantitation (or AQUA) by introducing known quantities of isotope-labelled peptides into biological samples to be analysed after in-gel or in solution digestion of proteins with proteases. This permits the relative quantification of unlabelled peptides. Absolute quantitation can be accomplished by classic isotope dilution measurements, where stable isotope-labelled peptides are used to generate a standard curve.

Another example of in vitro labelling of proteins and peptides uses isotopic or isobaric chemical tags, e.g., iCAT, iTRAQ, TMT or isotope dimethylation reagents to create internal reference peptide standards for relative quantitation.

Another example of in vitro labelling uses enzymatic methods for isotope labelling to add ¹⁸O isotopes to peptide carboxyl termini through tryptic digestion in ¹⁸O-labelled water.

In addition to these *in vitro* labelling methods, metabolic labelling methods have become increasing important due to that fact that isotope labelled proteins produced this way are more likely to share the same conformation and environment as the analyte proteins and thus provide a better control than in vitro labelled proteins. Two major themes have been developed: one to use metabolic labelling to produce isotope-labelled proteins or concatenated polypeptides (QConCat) using Escherichia coli (*E. coli*) or cell cell-free extracts (US patent: 20130034867). These heavy proteins can then be purified, spiked into biological samples and relative quantification can be determined.

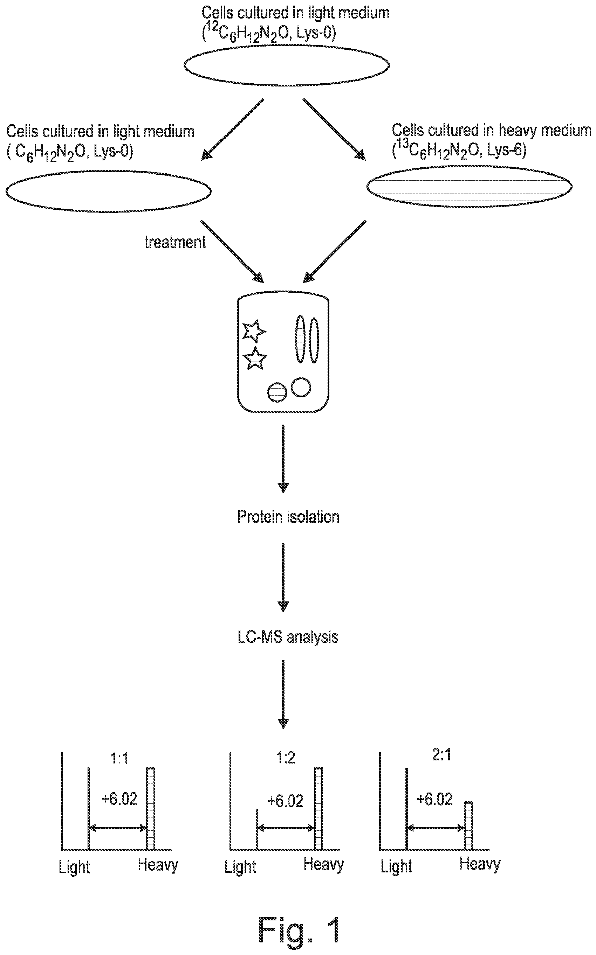

The other uses the whole cultured cells as an internal standard. Stable isotopes can be metabolically incorporated into proteins in cell culture (Stable isotope labelling by amino acids in cell culture, SILAC). SILAC methods use metabolic incorporation into proteins of heavy isotope-labelled amino acids or non-heavy isotope-labelled, i.e., unlabelled or light, amino acids. Typically SILAC labelling uses lysine and arginine, which in combination with trypsin digestion results in labelling of every peptide in the mixture (except for the c-terminal peptide of the protein). Typically it involves differential labelling of two or three sets of cells cultured in different stable isotope media: cells are grown with the natural amino acids, with ²D₄-lysine and ¹³C₆-arginine and with ¹⁵N₂¹³C₆-lysine and ¹⁵N₄¹³C₆-arginine (Figure 1). Sets of cells can be treated (with a drug or vehicle) after the complete (or nearly complete) incorporation of heavy amino acids during protein turnover. The two/three sets of cells are then combined, proteins are extracted and digested before liquid chromatography mass spectrometry analysis. The heavy amino acids create a distinct and known mass difference between the samples, and only affect the isotope distribution of the peptides in a minor and predictable way, thereby making data interpretation and quantification more accurate and robust. Peptides are quantified based on the ratio of ion currents of heavy and light pairs from the MS scans for each peptide across its elution time. Protein quantification is usually based on the median values of multiple peptides, resulting in high accuracy of ratio determination. While classical SILAC is based on cultured cells, this analytical strategy has more recently been demonstrated in multicellular organisms, animals, and to human tissues [3]. A conventional SILAC methodology is discussed in some detail in WO00/67017, the contents of which are incorporated herein by reference.

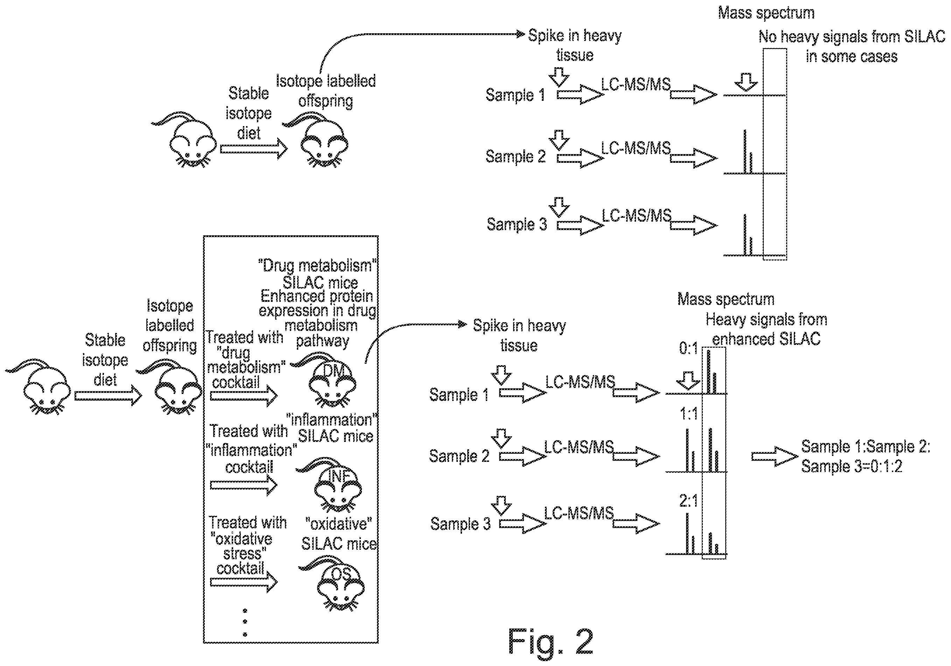

Such an *in vivo* stable isotope dilution design usually works well if proteins-of-interest express sufficient quantity in the heavy cells (or animals) since the accuracy and precision provided by stable isotope dilution relies on reliability of signals from the internal standards. In a scenario where the reference internal standards are not expressed, or are poorly expressed, the correction to heavy signals becomes impossible or unreliable and therefore accuracy of data is massively reduced. In biological systems, this has important ramifications as biologically or pathologically important proteins are often expressed at a very low level under unstressed or "normal" conditions. An example is illustrated in Figure 2A where stable isotope labelled liver samples were used to spike into control and two experimental (or treated) samples. For those peptides with no or little expression in heavy liver, the comparison between control to treated animals becomes difficult. One potential solution is to generate isotope labelled "disease" models (e.g. the combination of multiple culture cell lines to permit so-called "super SILAC"). This is a time consuming and expensive task which has limited the use of this powerful technique.

Methods that can enhance isotope protein expression in "normal" cells, organisms, tissues or animals (e.g. stable isotope labelling in mammals or SILAM), e.g. in a pathway specific manner for specific disease areas or applications, would be highly desirable.

WO 2011/160045, discloses SILAC approaches in animals, and exemplifies SILAC use in mice. WO 2009/058307 discloses in vivo isotopic labelling methods for quantitative glycomics. ZANIVAN SARA et al. ("In vivo quantitative proteomics: the SILAC mouse." METHODS IN MOLECULAR BIOLOGY, 2012, vol. 757 , 1 January 2012, pages 435- 450) discusses the production of SILAC mice.

This invention provides methods to enhance labelled protein expression by treating cultured cells, cell-free systems, cultured tissue, organisms, or animals with one or more agents targeting a specific or multiple pathways that are not activated, and hence show low protein expression, under normal conditions.

### Statements of the Invention

In a first aspect of the present invention, there is provided a method for preparing a reference sample of biological material for use in mass spectrometry-based protein detection or analysis, the method comprising:
a) providing a protein expression system;
b) providing said protein expression system with a nutrient composition comprising a source of amino acids or components for the synthesis of amino acids, wherein at least a portion of said amino acids or components for the synthesis of amino acids are labelled with a non-radioactive or radioactive isotope;
c) administering to said protein expression system a modulatory composition, wherein said modulatory composition increases abundance of one or more proteins in the protein expression system;
d) allowing said protein expression system to incorporate components from the nutrient composition into proteins during translation.

The present invention thus provides for the up-regulation of proteins of interest in the protein expression system, whereby those proteins of interest will incorporate appropriately labelled amino acids. Such reference samples can then be used in mass-spectrometry based methodologies where, in conventional techniques, quantification of the proteins of interest would be highly problematic because of the low expression levels of the protein of interest.

The term 'reference sample' in the present context means any sample containing biological material which is useful for a mass spectrometry-based protein analysis methodology. The reference sample can be an intact organism, e.g. a microorganism, an animal or plant. Alternatively said reference sample could be a tissue, an organ or cells derived from a plant or animal. Said reference sample could be a cell extract from cells of a microorganism, an animal or a plant.

The term 'protein expression system' is intended to refer to any biological cell, biological tissue, an organ, a non-human organism, a collection of non-human organisms, a portion of an organism, and a cell-free biological mimetic system. The term includes unicellular microorganisms (e.g. bacteria, archaea, fungi, and protozoa) and multicellular organisms (e.g. plants and non-human animals), or portions of such multicellular organisms. In a particularly preferred embodiment of the invention, the protein expression system is a non-human animal, especially a non-human mammal, and most preferably a mouse or rat.

The invention is applicable to any protein expression system in which the administration of a modulatory composition can affect protein expression levels. It will be obvious to the skilled person that this can be readily achieved in cells or animals using a wide range of agents. In the case of cell-free biological mimetic system modulation of protein expression can be achieved by administering modulators which target, for example, nuclear receptors. Exemplary cell-free expression systems (also known as an in vitro expression system) include the various 'In Vitro Protein Expression Kits' available from Thermo Scientific, which are based on *E. coli,* rabbit reticulocyte, wheat germ, insect or human cells. For example, the 'Thermo Scientific Pierce Human In Vitro Protein Expression System' allows for expressing proteins from DNA or mRNA templates in a cell-free solution containing essential components of the cellular translational machinery; extracts of an immortalized human cell line provide the ribosomes, initiation and elongation factors, tRNAs and other basic components required for protein synthesis.

'Non-radioactive isotope' in the present context includes stable isotopes of atoms contained in conventional biological molecules, and includes nitrogen-15, carbon-13, oxygen-17, oxygen-18, sulphur-34, selenium-74, selenium-76, selenium-77, selenium-78, selenium-82 and hydrogen-2. Non-radioactive isotopes are preferred. Radioactive isotopes may also be used in some cases, but are not preferred because of the difficulties the use of such isotopes present.

'Nutrient composition' in the present context relates to a composition which is provided to the protein expression system and which includes amino acids or amino acid precursors which the protein expression system will use during protein synthesis. Accordingly, amino acids or amino acid precursors in the nutrient composition, which include labelled amino acids or amino acid precursors, are incorporated into proteins generated by the protein expression system. The nutrient composition may or may not include other nutrients to support the protein expression system, e.g. a suitable energy source, vitamins, minerals, cofactors, etc. The nutrient composition may be the sole medium or diet provided to the protein expression system, or it can be combined with additional compositions. It is also possible that the nutrient composition may comprise other labelled molecules, e.g. to permit labelling of biomolecules such as carbohydrates or lipids.

In the case of cells, tissues or cell-free biological mimetic systems, the labelled nutrient composition suitably comprises a medium (e.g. a culture medium) in which the cells or tissues are grown.

In the case of animals, the nutrient composition suitably comprises a diet, i.e. food and/or drink, administered to the animal.

Preferably said nutrient composition is the sole source of amino acids or components for the synthesis of amino acids available to said protein expression system.

Preferably at least 90%, more preferably at least 95%, at least 98%, at least 99% or substantially 100%, of at least one amino acid present in said nutrient composition is labelled. Preferably said labelled amino acid is an essential amino acid for the relevant protein expression system.

Preferably said labelled amino acid is one or more of ²D₄-lysine, ¹³C₆-arginine, ¹⁵N₂¹³C₆-lysine and ¹⁵N₄¹³C₆-arginine. However, other labelled amino acids or alternative non-natural amino acids could of course be used. Preferably the labelled amino acids are selected such that they will segregate reliably when a protein sample is subjected to proteolysis during future analysis, e.g. for mass spectrometry. Labelled lysine and arginine are ideally suited to use with trypsin, which cleaves peptide chains mainly at the carboxyl side of the amino acids lysine or arginine.

Optionally the nutrient composition can be modified so that almost all, or all, of any element is replaced by a labelled form. The particular level of enrichment may depend on the isotope chosen. For example, a medium enriched in nitrogen-15 to between 90%-100% is preferred, with 100% enrichment being most preferred. Because hydrogen-2 can be toxic, less than 100% hydrogen-2 is preferred. Isotopic depletion may also be used wherein less than the naturally occurring abundance of an isotope is provided in the medium.

Methods of non-radioactive or radioactive isotope labelling of cells and animals are well known in the art, and there is no need to recite in detail all the details of such methods:
- Labelling of cells for the purposes of SILAC is discussed in detail in Ong SE, Mann M: A practical recipe for stable isotope labeling by amino acids in cell culture (SILAC). Nat Protoc 2006, 1(6):2650-2660 and Ong et al.: Stable isotope labeling by amino acids in cell culture, SILAC, as a simple and accurate approach to expression proteomics. Mol Cell Proteomics 2002, 1(5):376-386.
- Labelling of mammals for the purposes of SILAM is discussed in detail in Rauniyar, N. et al., Stable isotope labeling of mammals (SILAM) for in vivo quantitative proteomic analysis. Methods 61, 2013, 260-268 and McClatchy et al. Differential Proteomic Analysis of Mammalian Tissues Using SILAM PLoS ONE, January 2011 | Volume 6 | Issue 1 | e16039.

The 'modulatory compositions' of the present invention can comprise one or more agents which acts upon the protein expression system to increase the abundance of one or more proteins of interest in the protein expression system. This is of enormous benefit for methods of SILAC or SILAM, where calculating the abundance of a protein in a test sample is often rendered impossible or highly inaccurate because there is insufficient abundance of the labelled reference protein to allow meaningful comparison with the test sample.

The modulatory composition can be administered to the protein expression system at any time suitable to induce increased protein abundance in the protein expression system so it is present at the required level in the reference sample of biological material. The modulatory composition can, for example, be administered for a period of from 1 minute to 1 year, more typically for a period of from 5 minutes to 10 days. The duration of application of the modulatory composition will of course depend on the type of protein expression system and the type of proteins to be induced. For example effects would be expected to be relatively rapid in a microbe, but are likely to be slower in a mammal, such as a mouse. Additionally, one would expect certain proteins to rapidly increase in abundance, e.g. cytosolic proteins of short half-life, whereas others, e.g. structural proteins, may take longer. Thus no definitive period for administration of the modulatory compositions can be defined for all circumstances. However it is straightforward for the person skilled in the art to test how long a modulatory composition must be applied for to achieve the desired effects. In the case of mice administered with the compositions described below, a period of 3 days or so was satisfactory. Periods of administration can be continuous or discontinuous.

In certain situations the modulatory composition can be administered after a sample of biological material has been obtained from the protein expression system. For example, in the case of an animal, a sample of cells or tissue may be removed from the animal. This sample can then be subjected to the modulatory composition in a suitable cell or tissue culture system. The media present in the culture system preferably includes isotope labelled amino acids, or components for the synthesis of amino acids, to ensure that any newly synthesised proteins include the isotope.

It is well-known that administration of a wide range of active agents to cells, tissues, animals and plants elicits changes in protein expression. The present invention harnesses this in order to provide a reference sample of any protein expression system in which the abundance of one or more proteins to be studied have been increased.

In a preferred embodiment of the present invention, a modulatory composition comprising one or more modulating agents are administered in order to increase expression of proteins in one or more pathways, i.e. an inducer or inducing agent. This results in higher abundances of labelled proteins in the protein expression system, and means that samples of such modulated cell expression systems can be used as labelled references to determine relative abundances in test samples.

Such pathways can be disease pathways, metabolic pathways, signalling pathways or the like. Biological pathways are often extremely complex and many remain incompletely understood. However, that is of no consequence in the present invention as all that is required is that up-regulation of proteins of interest is achieved.

Exemplary pathways which can be targeted in the present invention include:
- Drug metabolism;
- Inflammation/immune system;
- Oxidative stress;
- Cell-cycle regulation;
- Blood clotting;
- Complement;
- DNA Damage
- Autophagy
- Heat shock
- ER stress
- Diabetes/Obesity
- Neurological/psychological diseases
- Cholestatic Liver Disease

Table 1 shows several exemplary receptors and corresponding agents which can be used to target them and thereby modulate expression of various downstream proteins. Table 2 shows several exemplary disease pathways or pathways that are associated with diseased and corresponding agents which can be used to induce such pathways.

**Table 1 - Examples of endogenous hormones, bile acids, xenobiotic compounds and therapeutic drug for inducing receptors.**

| ***Targeting Receptor*** | ***Inducing Agents*** |
|---|---|
| **Orphan receptors** | |
| Constitutive androstane receptor (CAR) | - barbiturates e.g. phenobarbital and TCPOBOP; |
| | - steroids such as androstanol |
| | - TITCO |
| Pregnane X receptor | - steroids, e.g. pregnenolone 16 alpha carbonitrile, glucocorticoids (e.g. dexamethasone); |
| | - Antibiotics, e.g. rifampicin |
| | - Meclizine dihydrocholride |
| | - RS12813 |
| Liver X receptor | - LXR like receptors agonists: Oxysterols, Fexaramine, GW3965, GW4064, T0901317 and their analogues |
| Farnesoid X receptor | - bile acids, e.g. lithocholic acid and chenodeoxycholic acid |
| Retinoid x receptor | - Agonists such as AC261066, AC55649, Adapalene, AM580, AM80, BMS753, BMS961, CD1530, CD437, Ch55, Retinoilc acid, Tazarotene, retinoids (e.g.TTNPB). |
| Peroxisome proliferator-activated receptor | - clofibrate, |
| | - thiazolidinediones, e.g. rosiglitazone, |
| | - fatty acids |
| | - prostaglandins |
| | - PPARα agonists: CP775146, fenofibrate, GW7647, Oleylethanolamide, Palmitoylethanolamid, Wy14643. |
| | - PPARδ agonists:GW0742 and L165.041 |
| | - PPARγ agonists: Ciglitazone, GW1929, LG100745, nTZDpa, Pioglitazone, 15 deoxy-Δ-12,14-prostaglandin J2, S26948, Troglitazone |

| **Endocrine receptors** | |
|---|---|
| Estrogen receptor | - Estrogen receptor agonists such as Daidzein, Diarylpropionitrile, DY131, ERB041, oestrogens, FERb033, GSK4716, Liquiritigenin, PPT WAY200070 and their analogs. |
| | - tamoxifen |
| Glucocorticoid receptor | - Cortisol, selective glucocorticoid receptors such as Mapracorat, ZK216348 and 55D1E1, Ru24858, and octahydrophenanthrene-2,7-diol derivative |
| Mineralocorticoid receptor | - Aldosterone |
| | - Mineralocorticoid receptor agonists such as fludrocortisone. |
| Progesterone receptor | - Progesterone, selective progesterone receptor modulators such as Ulipristal acetate, Asoprisnil, CDB-4124 |
| Androgen receptor | - Androgen receptor agonists such as CI-4AS-1, Testosterone, |
| Vitamin D receptor | - Vitamin D receptor agonist such as calcitriol, doxercalciferol, EB1089, Ercalcitriol, Califediol, 22-oxacalcitriol, Tacalcitol, vitamin D, Alfacalcidol |
| Retinoic acid receptor | - Agonists such as AC261066, AC55649, Adapalene, AM580, AM80, BMS753, BMS961, CD1530, CD437, Ch55, Retinoilc acid, Tazarotene, retinolic acid analogs (e.g.TTNPB). |
| Thyroid hormone receptor | - thyroid hormone, thromimetics such as GC1. |

| **Other receptors and inducible transcription factors** | |
|---|---|
| Aryl hydrocarbon receptor | - agonists such as ITE, MeBIO, Pifithrin-a hydrobromide, L-kynurenine |
| | - beta-naphthoflavone; |
| | - polychlorinated biphenyls, e.g. Aroclor 1254; |
| | - dioxins, e.g. TCDD |
| Nuclear factor E2-related factor 2 | - Nrf-2 pathway activators such as Dimethyl fumarate, mono-methyl fumarate, TAT14 peptide and their derivatives, |
| | - Ethoxyquin; |
| | - butylated hydroxyanisole; |
| | - triterpinoids, e.g. TBE31; |
| | - 4-hydroxynonenal; |
| | - sulforaphane |
| Activator protein-1 | - phorbol myristate acetate |
| Selective liver receptor homolog-1 (LRH-1) | - DLPC |
| Thyroid receptor | - GC1 |
| Broad spectrum steroid receptor ligands | - Guggulsterone |

**Table 2 - Examples of endogenous hormones, bile acids, xenobiotic compounds and therapeutic drug for inducing expression pathways or disease pathways.**

| ***Pathways*** | ***Inducing Agents*** |
|---|---|
| **DNA Damage** | - platinum compounds, e.g. cisplatin; |
| | - alkylating agents, e.g. cyclophosphamide, 1,3-propane sultone, and carmustine; |
| | - mitomycin C; |
| | - UV irradiation |
| **Autophagy** | - nutrient starvation; |
| | - calcium chloride; |
| | - thioacetamide |
| | - mTOR inhibitors such as rapamycin and analogs (such as CCI-779), torin |
| | - Ca2+ channel blockers such as perhexiline, amiodarone, nicolsamide , rottlertin, verapamil, loperamide, amiodarone, nimodipine, nitrendipine, niguldipine, nicardipine and pimozide) |
| | - PI3 kinase inhibitors (e.g. PI103 and phenethyl isothiocyanate) |
| | - Dexamethasone |
| | - Calpain inhibitors (e.g. calpastatin) |
| | - Histone acetyl transferases inhibitor |
| | - Resveratrol |
| | - Dopamine antagonists (e.g. fluspirilene and trifluoperazine) |
| | - SMERs |
| | - Dorsomorphin |
| **Heat shock** | - Heat; |
| | - 4-hydroxynonenal; |
| | - Acrolein |
| **Inflammation**/**immune system** | - cytokines, e.g. TNFalpha; IL-1; IL6 |
| | - chemokines; |
| | - LPS; |
| | - cigarette extract; |
| | - Hemocyanin (e.g. KLH) |
| **ER stress** | - Cholesterol; |
| | - Thapsigargin; |
| | - Tunicamycin; |
| | - brefeldin A; |
| | - ionophore A23187; |
| | - Hypoxia inducers such as cobalt. |
| **Phase II drug metabolism** | - Phase 2 drug metabolism inducer such as sulforaphane, Oltipraz |
| | - NFkB activators such as gram-negative bacterial endotoxin lipopolysaccharide (LPS), and TNF-α and IL-1β. |
| **Diabetes/Obesity** | - High fat diet |
| | - insulin |
| **Neurological/psychological diseases** | - psychotropic agents, e.g. alcohol, ketamine, phencyclidine |
| **Cholestatic Liver Disease** | - antibiotics; |
| | - non-steroidal anti-inflammatory drugs (NSAIDs) |
| **Neuronal toxicity** | - 6-Hydroxydopamine |
| **Cell reprogramming** | - DZNep, ALK5 inhibitor SB431412 and MEK inhibitor PD0325901, Thiazovivin, microRNA, Acyclic retinoid, AKR1B10 inhibitors such as Zopolrestate, albumin, FGFs (e.g. FGF2) |

Accordingly, the modulatory composition of the present invention can comprise one or more of the agents set out in Tables 1 and 2.

In a preferred embodiment of the invention, the modulatory composition is administered to induce expression of proteins involved in drug metabolism (e.g. P450 isoforms), inflammation or oxidative stress.

To increase expression of proteins involved in drug metabolism, suitably the modulatory composition comprises one or more of TCPOBOP or rafamycin, pregnenolone 16α-carbonitrile (PCN), and ethoxyquin. Suitably the composition comprises TCPOBOP, which has been shown to efficiently increase levels of the low-abundance cytochrome protein Cyp2B10.

It would be routine for the person skilled in the art to select additional or alternative modulatory agents. Any potential modulator of interest can be screened against its effects on expression of proteins of interest. A suitable methodology for screening one or more agents is described in Examples 1 and 3 below. The agent (or a combination of agents) is administered to an organism and the effects on protein abundance of proteins of interest can be examined using conventional techniques such as western blot or ELISA. qPCR can be used to examine effects on mRNA levels, which are of course indicative of increased expression. It is worth noting at this point that increased protein abundance need not result from increased expression, but could also result from a decrease in protein degradation, e.g. via the ubiquitin pathway. Thus the modulators of the present invention can increase expression and/or increase longevity of the proteins of interest.

A method for the selection of a modulatory composition for the present invention and its use in the present invention can comprise one or more of the following steps:
1. Select a pathway of interest;
2. Select potent inducer or modulator compounds for each pathways and define dosage based on literature;
3. Carry out single or combined treatments to single cell organisms, cells or animals;
4. Examine protein expression in targeted pathways by LC-MS/MS (followed by database search and comparison of signal intensity) or by Western blotting;
5. Define the optimised modulatory composition based on these results. An ideal composition will typically have optimal levels of enhancement in highest number of proteins in the selected pathways;
6. Treat stable isotope labelled single cell organisms, cells or animals with the defined composition;
7. Examine protein expression in the treated single cell organisms, cells or animals by LC-MS/MS followed by appropriate database search;
8. Define the characteristics of peptides for protein(s) of interest (i.e. m/z of precursor pairs (heavy and light), transitions (for triple quadrupole analysis), retention time, etc.);
9. Spike in the heavy sample to experimental samples in experiments; and
10. Monitor the intensities of each peptide pairs and calculate the ratios.

The modulatory composition can be administered in any suitable way. For cells or tissues in culture, it can be in the culture medium. For animals it can be delivered orally (e.g. via food/drink), by injection (e.g. i.v., i.m. or i.p. injection), via inhalation, etc.

There is also provided a reference sample of biological material obtained or obtainable by the methods described above.

Suitably said reference sample of biological material is characterised in that at least 90%, more preferably at least 95% and especially at least 98%, of all the proteins in the protein expression system are labelled with the non-natural isotope.

Suitably said reference sample is an intact organism, e.g. a microorganism or an animal or plant. Alternatively said reference sample could be a tissue, an organ or cells derived from a plant or animal. Said reference sample could be a cell extract from cells of a microorganism, an animal or a plant.

Said reference sample comprises one or more proteins that have been induced to a higher abundance than a control sample which has not been exposed to a modulatory composition. Preferably said one or more proteins have been increased by at least 100% or more compared to a control which has not been exposed to the modulatory composition.

According to a further aspect of the present invention there is provided the use of a reference sample of biological material produced by the methods described above in mass spectrometry-based protein analysis. For example the reference can be used in a SILAC or SILAM methodology to analyse relative abundance levels of one or more proteins of interest.

Thus, in one embodiment the present invention provides a method for determining the relative abundance of one or more proteins of interest in samples of biological matter, the method comprising:
a) providing a first protein expression system;
b) providing a second protein expression system;
c) applying a test condition to at least one of said first or second protein expression systems;
d) providing a non-radioactive or radioactive isotope-labelled reference sample comprising an equivalent protein expression system to those in a) and b), wherein said reference sample is obtained by the methods described above;
e) obtaining a first sample from the first protein expression system;
f) obtaining a second sample from the second protein expression system
g) combining at least a portion of the first sample with a portion of the reference sample to form a first combined sample;
h) combining at least a portion of the second sample with a portion of the reference sample to form a second combined sample;
i) isolating or enriching one or more proteins from each of said combined samples;
j) subjecting the isolated or enriched proteins protein to mass spectroscopy to develop a mass spectrum;
k) computing a ratio between the peak intensities of at least one pair of closely spaced peaks;
l) determining the relative abundance of a protein in each sample compared to the reference sample based on the at least one computed ratio; and optionally
m) identifying the protein.

These steps describe conducting a SILAC or SILAM protocol in general terms. It will be noted that the reference sample of the present invention is not typically subjected to the relevant test conditions to which the first and second test sample are subjected (though in certain situations it could be). The reference sample fulfils the role of a set reference standard to against which both the first and second samples can be compared. The abundance of the relevant protein in the first sample can be compared against the labelled protein in the reference sample, and a first ratio calculated. The abundance of the relevant protein in the second sample can be compared against the labelled protein in the reference sample, and a second ratio calculated. These two ratios can then be compared. Because both ratios are calculated against an identical reference, the first and second ratios can be directly compared to measure changes in protein abundance in the first and second samples. Because the reference standard has been manipulated to increase the amount of the relevant protein of interest, the amount of labelled protein is significant enough that a meaningful ratio can be calculated.

Accordingly, it is preferred that the method is performed to analyse the abundance of one or more proteins which are insufficiently expressed for conventional SILAC, SILAM or related methodologies to work effectively.

Steps k) to l), and optionally m) can of course be repeated for one or more additional proteins of interest.

Preferably the method comprises digesting the at least one protein of interest with a protease. Suitably the protease is trypsin. Trypsin is preferred because it cleaves precisely at the sites of lysine and arginine, yielding doubly-charged peptides which typically have a length of from about 5 to 50 amino acids and a molecular weight of between about 700-5,000. Such peptides are particularly appropriate for analysis by mass spectroscopy, especially by electrospray ionization mass spectroscopy. Other site specific proteolytic enzymes which may be used include Ly-C, Asp-N and Glu-C, for example. Pepsin, subtilisin and proteinase 1c are low specificity enzymes which may also be used. Chemical reagents may also be used to digest the proteins. For example, cyanogen bromide may be used to cut a protein into peptides at the site of methionine. BNPS-skatole may be used to cleave at the site of tryptophan. Acid hydrolysis may also be used.

This digestion step generates a plurality of peptide fragments from the protein which can then be analysed via the well-known techniques of peptide mass fingerprinting. Accordingly, the method suitably comprises identifying the protein of interest from the mass spectrum.

The proteins or digested proteins can therefore be subjected to mass spectroscopy. Any suitable mass spectrometer may be used to analyze the peptides or proteins. For example, the mass spectrometer may be a Matrix-Assisted Laser Desorption/Ionization ("MALDI") Time-of-Flight ("TOF") Mass Spectrometer, available from PerSeptive Biosystems, Framingham, Mass. and Agilent, Santa Clara, California; an Electrospray Ionization ("ESI") ion trap-Orbitrap hybrid mass spectrometer, available from Thermo Scientific, San Jose, California; or an FTICR mass spectrometer, available from Thermo Scientific or Bruker, Bremen, Germany.

The protein or proteins subjected to the mass spectroscopy are preferably identified. The identification step can take place at any time after mass spectrometric analysis of a protein or peptide mixtures. Protein identification software which uses algorithms to compare the mass spectrum with theoretical MS and MS/MS information of peptides in a database of proteins are available. One such algorithm, ProFound, uses a Bayesian algorithm to search protein or DNA databases to identify the optimum match between the experimental data and the protein in the database. W. Zhang, B. T. Chait, "Proceedings of the 43rd ASMS Conference on Mass Spectroscopy and Allied Topics," Atlanta, Ga. (1995) p. 643. ProFound may be accessed on the World-Wide Web at <http//prowl.rockefeller.edu> and <http//www.proteometrics.com>. Profound accesses the non-redundant database (NR).

Alternative algorithms for protein identification include:
- Mass Search (http:/lcbrg.inf.ethz.ch/subsection 3-3.html);
- MOWSE (http://www.seqnet.dl.ac.uk//mows.html);
- MSFIT (http://prospector.ucsf.edu/ucsfhtml/msfit.htm);
- Peptide Mass Search (http://www.mdc-berlin.de/emu/peptide_mass.html); and
- Peptide Search (http://www.mann.embl_heidelberg.de/services/peptide search/fr_peptide searchform.html).
- Mascot (http://www.matrixscience.com/search_form_select.html)
- Proteome Discoverer (Thermo Scientifics, USA)
- Peaks (http://www.bioinfor.com/peaks/features/peaksdb.html)

See also, James, Peter, "Protein identification in the post-genome era: the rapid rise of proteomics", Q. Rev. Biophysics, Vol. 30, No. 4, pp. 279-331 (1997), which is incorporated by reference, herein.

The method may alternatively comprise identifying the protein by electrophoresis, using antibodies (e.g. via ELISA or Western blot) or by a bioassay (e.g. an activity assay).

Suitably step i) comprises extracting proteins from a combined sample, and can comprise separating the extracted proteins by a process chosen from the group consisting of:
- one-dimensional electrophoresis,
- two-dimensional electrophoresis,
- ultracentrifugation,
- chromatography, and
- affinity binding.

The method of the present invention thus can comprise:
- removing a plurality of proteins of interest from the combined samples;
- optionally digesting the plurality of proteins of interest into a plurality of peptides;
- subjecting the digested or undigested proteins to mass spectroscopy to develop a mass spectrum;
- selecting a plurality of pairs of closely spaced peaks on the mass spectrum;
- computing the ratio of the intensities of the peaks in each pair;
- determining the protein from which the pair of peaks in the mass spectrum are derived based on the mass spectrum; and
- determining the relative abundance of the protein in each sample compared with the reference sample.
The method can optionally comprise determining the relative quantity of a modified protein of interest in each sample. For example, the type of peptide modification can be chosen from the group consisting of phosphorylation, glycosylation, and acylation of the peptide. The determining step can comprise determining the difference in the relative abundance of the modified peptide in each sample. The method can further comprise determining the site of the modification on the protein.
Suitably step c), i.e. exposing one of the protein expression systems to a test condition, comprises subjecting one of the said first and second protein expression systems to an environmental or chemical stimulus.

For example, where the first and protein expression systems are non-human organisms and step c) comprises genetically manipulating one of the said first and second non-human organisms.
The genetic manipulation can comprise performing gene therapy.
Alternatively, step c) can comprise subjecting the one of the said first and second protein expression systems to a treatment chosen from the group consisting of a virus, a bacteria and a carcinogen.
Suitably the protein of interest is a marker for the effect of the modulating step on a biological process.
It is, of course, highly desirable that the first and second protein expression systems are identical, to ensure that the relevant test condition is responsible for any changes in protein abundance. Furthermore it is desirable that the reference sample comprises or is derived from the same protein expression system as the first and second protein expression systems. For example, suitably the first and second protein expression systems and the reference sample are all mice or mouse-derived tissues or cells.

'Equivalent' in the context of the protein expression systems of the present invention means that the protein levels in the reference and the test sample(s) can be meaningfully compared. Typically this means that the proteins of interest will have essentially the same sequences, preferably identical sequences. Typically equivalent protein expression systems will be of the same species, more preferably of the same strain/variety. Suitably the equivalents can be siblings, clones or otherwise closely related.

Suitably the first protein expression system is a control, and the second is subjected to the relevant test condition. Both the control and the test are then compared against the same reference so that relative protein abundances in the control and the test can be compared.

### Brief Description of Figures

Figure 1 - shows an illustration of a typical SILAC workflow (taken from reference [1]).
Figure 2 - shows. (A) Traditional SILAM design. (B) Pathway-enhanced SILAM design, i.e. it shows generation of isotope mice with enhanced protein expression at specific pathways and their use in a SILAM system.
Figure 3 - shows a chart comparing abundance of various proteins of drug metabolism pathways in cells induced by TCPOBOP and pregnenolone 16α-carbonitrile (PCN) versus a corn oil control.
Figure 4 - is an image of a gel showing increase of protein expressions in drug metabolism pathways by one or more inducers.
Figure 5 - shows mass spectra which demonstrate that "drug metabolism" pathway-enhanced SILAM improves the quantification of Cyp2B10 in mouse liver compared to the traditional SILAM.

### Specific Description of Embodiments of the Invention

In a known SILAC method, as illustrated in Fig 1, cells of the same origin are cultured in media containing Lys-0 or Lys-6 for several passages so that all Lys-0 in proteins of heavy cells (strip plate) are replaced with Lys-6. One of the sets can be treated whilst the other is used as a control. Samples of each of the cells can be combined normally in 1:1 ratio and proteins are isolated, digested with proteases (in this case LysC, but trypsin is often used) before being subjected to LC-MS/MS analysis. Because all the resultant peptides should have a lysine in the C-terminus, the gap between the light and heavy should be 6.02 amu (if it is a singly charged) or 3.01 (if it is a doubly charged) and so on. The ratio of ion currents of heavy and light pairs can be calculated for protein quantification.

More details of a conventional SILAC methodology are set out in WO00/67017, Ong SE, Mann M: 'A practical recipe for stable isotope labeling by amino acids in cell culture (SILAC)'. Nat Protoc 2006, 1(6):2650-2660, and Ong et al.: 'Stable isotope labeling by amino acids in cell culture, SILAC, as a simple and accurate approach to expression proteomics'. Mol Cell Proteomics 2002, 1(5):376-386, the contents of which are incorporated by reference.

In a known Stable Isotope Labelling in Mammals (SILAM) methodology (as illustrated in Fig 2A) mice are fed with stable isotope diets and proteins in the offspring from these mice will be labelled with isotope labelled proteins. The offspring mice are retained on a stable isotope diet to maintain high levels of labelled proteins. Tissues from these animals will be used as a reference standard for protein quantification in control and treatment groups. For some proteins where the heavy stable isotope signals are not detectable, or only show at very low levels, the comparison between control and treatment groups becomes difficult and unreliable. This is because where the abundance of labelled proteins is low, no corresponding peak is identified on the mass spectrum, or the peak is so small as to introduce massive inaccuracies. For this reason the ratio between peptides of the protein of interest and the peptides from the labelled reference tend to be infinite or zero (i.e. because the denominator is zero (or very close to it) or vice versa, respectively).

In embodiments of the present inventions (as illustrated in Fig 2B), referred to herein as 'pathway-enhanced' SILAM design, mice are likewise fed with stable isotope diets and proteins in their offspring from these mice will be labelled with isotope labelled proteins. Modulator compositions comprising one or more modulating agents targeting specific pathways are then administrated to animals to induce protein expression of these pathways (e.g. drug metabolism, inflammation, oxidative stress etc.). Thereafter the liver, for example, of such "drug metabolism" reference mice can be mixed 1:1 with liver samples (1 to 3) from both control and the two treatment groups. Fig 2b shows one control and two treatment groups. The control and treatment groups are not labelled.

The proteins (heavy, from the reference sample, and light, from the treatment and control) can be then extracted together, digested with protease (e.g. trypsin) and analysed in LC-MS/MS. The signals from heavy liver (shown in dark on the right of the mass spectrum) can be used as a reference for the signals from experiment groups (show to the left of the mass spectrum). In the example shown no expression of a drug metabolism protein is found in control animals (thus the ratio of light to heavy is 0), whilst Treated 1 has a ratio of 1 (i.e. equivalent abundance to the reference) and Treated 2 has a ratio of 2 (i.e. twice the abundance of the reference). The relative protein expression can be expressed as 0:1:2. In the absence of heavy signals provided by the reference sample, it would have been difficult to compare treated 1 and treated 2 - as illustrated by Fig 2A.

To generate the enhanced SILAM or SILAC reference samples from, for example, mice, the mice are fed with stable isotope diets containing only labelled (heavy) lysine and/or arginine (e.g. ¹³C₆ ¹⁵N₂ L-lysine-2HCl and/or ¹³C₆ ¹⁵N₄ L-arginine-HCl).

Proteins in the offspring after second generation (F2) from these mice are almost exclusively labelled with the non-natural isotope, i.e. to >98% (see Geiger T, Wisniewski JR, Cox J, Zanivan S, Kruger M, Ishihama Y, Mann M: 'Use of stable isotope labeling by amino acids in cell culture as a spike-in standard in quantitative proteomics'. Nat Protoc, 6(2):147-157).

A modulatory composition comprising one or more endogenous hormones or xenobiotic compound targeting specific pathways (for examples, see Table 1 and 2) is administrated to the animals to induce protein expression of these pathways (e.g. drug metabolism, inflammation, oxidative stress etc.). The modulatory composition should be administered for sufficient time for the relevant proteins to be induced and synthesised. The up-regulated proteins will be labelled with the non-natural amino acids. Tissues or cells from the mice can then be used as a reference standard in SILAC or SILAM experiments. Because of the up-regulation of the proteins of interest, they will perform far better as reference samples than conventional reference samples from known SILAM techniques in the case of low abundance proteins.

Referring once again to Fig 2B, the liver lysate of "drug metabolism" induced mice (i.e. mice which have been induced to increase the abundance of proteins in the drug metabolism pathway) is mixed 1:1 with liver samples from the control and treatment groups. The proteins (heavy to the right of the spectrum and light to the left of the mass spectrum) can then be extracted together, digested with protease and analysed in LC-MS/MS. In the absence of heavy signals for some poorly expressed proteins in traditional SILAM design, it would be either error-prone or impossible to compare the expression between the groups (Figure 2A). The similar principle can be applied to other pathways such as "inflammation SILAM" or "oxidative stress SILAM, and to other cells, organisms, and cell-free systems.

### Example 1 - An optimised "drug metabolism" pathway-enhanced SILAM model for preclinical studies.

In order to generate a drug metabolism-enhanced SILAC model, the protein expression profiles of liver from animals induced with TCPOBOP (1 mg/kg) or pregnenolone 16α-carbonitrile (PCN) or vehicle were examined. Wild type mice were treated with corn oil or 1 mg/kg TCPOBOP or pregnenolone 16α-carbonitrile (PCN) once daily by i.p. injection for 3 days. Animals were sacrificed 3 days after dosing, liver tissue excised and snap-frozen in liquid nitrogen for storage at -80°C.

Frozen liver tissue was thawed by addition of 10 volumes SDT lysis buffer (4% SDS, 0.1M DTT, 100 mM Tris-HCl pH7.6) then homogenised by rotor-stator (2 x 5 sec at 20k revolutions). Homogenate was heated to 95°C for 5 minutes, sonicated (2 x 5 sec), then centrifuged at 16,000 x *g* for 10 minutes. Supernatant (protein sample for analysis) was removed, aliquoted and stored at -80°C until use. Protein samples (total of 30 µg/well) were electrophoresed through a 12% bis-tris gel in MOPS running buffer supplemented with antioxidant (all Life Technologies, Paisley, UK) alongside a Spectra multicolour broad range protein ladder (Thermo Fisher Scientific, Waltham, MA). Gels were stained with coomassie blue, destained, and then rehydrated with Milli-Q water. Gel regions containing proteins of interest, as described in the results section, were removed with a clean scalpel, sliced finely (ca. 1 x 1 mm cubes) and added to 1.5 mL PCR-clean eppendorf tubes (Eppendorf). In-gel trypsin digest and peptide extraction was carried out according to the method of Schevchenko and colleagues[4]. Peptide sample concentration was determined by Nanodrop (Thermo Fisher Scientific) and adjusted to 0.2 mg/mL in water containing 0.1% (vol/vol) trifluoroacetic acid. A nanoflow liquid chromatograph (Agilent 1200) with a LTQ-Orbitrap XL (Thermo Fisher Scientific) was used to analyse the protein digests. Approximately 0.4 µg of total peptide was loaded onto a trap column at a flow rate of 10 µL/min for 3 min and the flow was then reversed to an Agilent Zorbex nano C18 column (0.0075 mm ID; 15 cm; 3 µm particle size). The peptides were resolved with a 3 hr binary gradient at a flow rate of 300 nL/min as follows: 0% Buffer B for 5 min followed by 2-30% Buffer B for 140 min; 30-90% Buffer B for 15 min; 90-0% Buffer B for 10 min and 0% Buffer B for 10 min. Buffer A contained 2% acetonitrile and 0.1% formic acid in water and Buffer B contained 0.1% formic acid in acetonitrile. The column was periodically cleaned with a 2 µL injection of buffer containing 50% acetonitrile and 0.1% formic acid in water. A Proxeon nanospray source with a stainless steel emitter (Thermo Scientific) was used to interface Agilent nanoLC and LTQ-Orbitrap. Spray voltage was set at 1.8kV. The Orbitrap was tuned using Glu-Fibrinogen B peptide. For the protein/peptide identification, an acquisition method that consisted of full scans between 300-2000 a.m.u (in Orbitrap) and data dependent MS/MS with top 6 precursor ions (2⁺ to 4⁺ charged) in LTQ was employed. Orbitrap was operated in a profile mode at the resolution of 30,000 or 60,000 with a lock mass set at 445.1200 (polycyclodimethylsiloxane [5]), LTQ was operated in a centroid mode with isolation width =1 (m/z); normalised collision energy = 0.25; and activation time = 30 ms. A dynamic exclusion of 30 sec was used to maximize the acquisition of MS2 on peptides with lower intensity. Data were analysed using SIEVE software in conjunction with Mascot database search engine.

The sum of all peptides for each protein of interest is normalised to total ion current and the relative intensity of each protein was compared between the three groups.

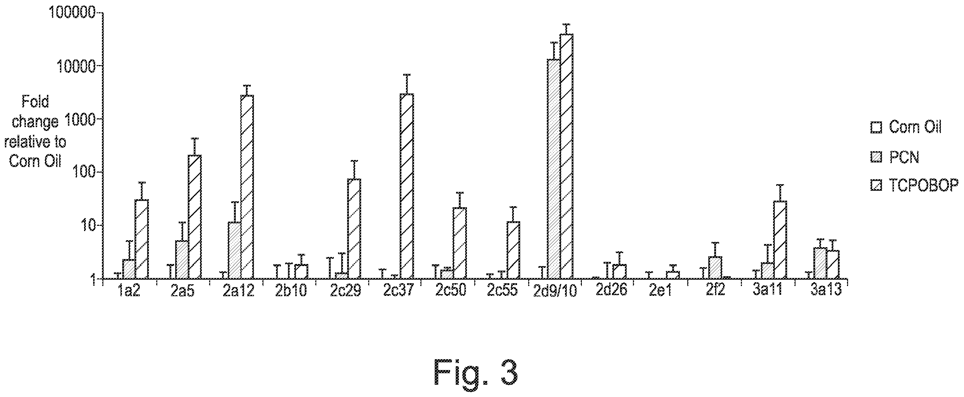

Many drug metabolism related proteins were found to be lowly expressed in wild type mice treated with corn oil, including Cyp1a2, 2a5, 2a12, 2b10, 2c29, 2c37, 2c50, 2c55, 2d9/10, 2d26, 2e1, 2f2, 3a11 and 3a13 (Fig. 3). We found TCPOBOP and to a lesser extent, PCN, significantly increased the expression of many of these proteins (but not all) relative to the total ion current. We recognised an ideal drug metabolism pathway enhanced model requires the majority of drug metabolism enzymes to be within a detectable range. A further refinement of a cocktail inducer was carried out.

As an example of the procedure to optimise a modulatory composition for the maximum expression of proteins in the "drug metabolism" pathways, several inducers for constitutive androstane receptor (CAR), e.g. TCPOPOP, pregnane X receptor (PXR), e.g. PCN and rifampicin, and others were systematically tested.

Wild type mice (C57BL/6) were treated with corn oil or 1 mg/kg TCPOBOP or rafamycin, pregnenolone 16α-carbonitrile (PCN) or a combination of these drugs once daily by i.p. injection for 3 days and the expression of drug metabolism enzymes were analysed using Western blot. The cocktail treatments induced much higher expression of constitutively lowly express proteins compared to single compound alone.

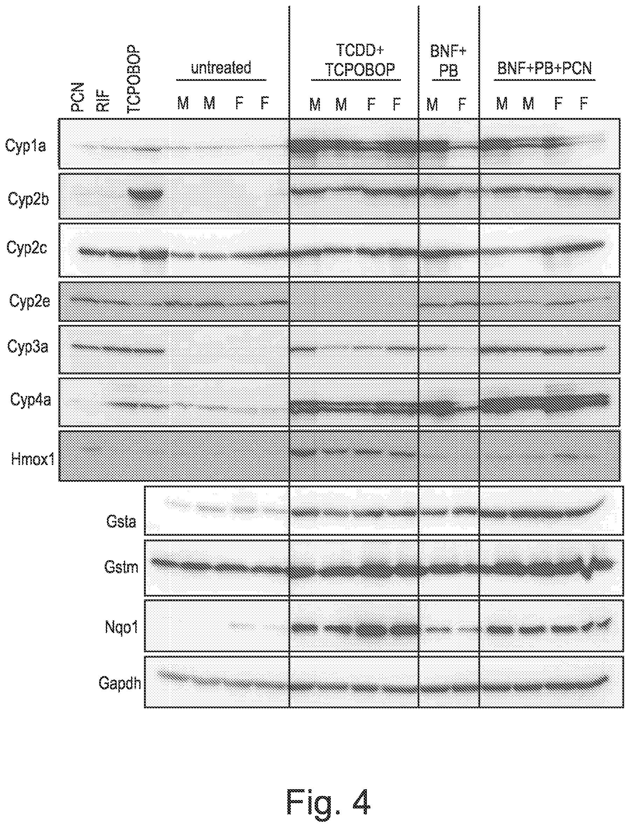

The induction of several "drug metabolism" proteins by single and multiple inducers in CAR and PXR pathways are demonstrated in Fig. 4. Stable isotope labelled wild type mice (C57BL/6, 4 months old) were treated with corn oil or 1 mg/kg TCPOBOP or rafamycin (RIF), pregnenolone 16α-carbonitrile (PCN) or a combination of these drugs once daily by i.p. injection for 3 days and the expression of drug metabolism enzymes were analysed using Western blot with antibody known to have proven specificity. In short, liver proteins were extracted using a lysis buffer containing 2% SDS. The protein lysate was resolved in a 4-12% SDS-PDGE followed by electrotransfering to a PVDF membrane. After washing with phosphate buffer saline and blocking with 5% skimmed milk, the membrane was incubated with the primary antibody against the targeted proteins shown in Figure 4 for 8 hours at 4 °C. The membrane was then washed twice with phosphate buffer saline and incubated with a horseradish peroxidase coupled secondary antibody against the primary antibody. The signals were detected using enhanced chemiluminescence system in conjunction with X-ray film.

We found that PCN induced a noticeable Cyp3a protein expression but no change to other Cyp proteins examined whilst RIF treatment alone increased Cyp3a and 4a expression. Treatment with TCPOBOP enhanced significantly the expression of many proteins examined except for Cyp2e and Homx1. The induction of Cyp1a and 4a by TCPOBOP was considered moderate.

Many drug metabolism enzymes including Cpy1a, Cyp2b, Cyp3a, Cyp4a and Nqo1 are expressed at a very low level in untreated mice, and therefore this was a sensible experimental model to exemplify the potential of the present invention. Dramatic increases of many of these enzymes are observed in mice treated with a combination of modulatory agents, whereas some specific increases were found in single drug treatments.

The results suggest that a modulatory composition comprising a 'cocktail' of modulatory agents can dramatically induce the expression of key drug metabolism enzymes in multiple nuclear receptor pathways.

### Example 1B - Variation of "drug metabolism" pathway-enhanced SILAM model

As a variation of the test described above, an optimised modulatory composition comprising pregnenolone 16α-carbonitrile (PCN), phenobarbital (PB) and beta-naphthoflavone (BNF) was administered to wild-type SILAM (i.e. labelled) mice for three days. Liver tissue was harvested and protein was extracted using buffer containing 2% SDS. The extracted liver protein was resolved in a 4-20% SDS-PAGE and the gel was stained with coomassie blue G250. Three molecular weight regions corresponding to 20-40kDa, 40-70kDa and 80-120kDa were cut and the three individual gel bands were further cut into 1 x 1 mm gel pieces before further washes with 100mM ammonium bicarbonate and dehydration using acetonitrile. In gel digestion was performed using 20ng/ml trypsin in 50mM ammonium bicarbonate with 10% acetonitrile at 37oC for 15 hours. In total, 42 more drug metabolism related proteins (19 in Phase 1, 12 in Phase 2 and 11 in Phase 3) were detected in the "pathway enhanced" liver than in the standard SILAM liver (see Table 3). Moreover, many of these proteins are considered to be key mediators of drug-drug interaction, and an appreciation of their levels is crucial for preclinical research in drug development.

**Table 3 - List of drug metabolism enzymes/transporters that can be quantified by "Drug metabolism" enhanced SILAM but not traditional SILAM.**

| **Phase 1** |
|---|
| Aldh1a7 Aldehyde dehydrogenase, cytosolic 1 |
| Cyp1a1 Cytochrome P450 1A1 |
| Cyp1a2 Cytochrome P450 1A2 |
| Cyp2a12 Cytochrome P450 2A12 |
| Cyp2a5 cytochrome P450 2A5 |
| Cyp2b10 cytochrome P450 2B10 |
| Cyp2c29 Cytochrome P450 2C29 |
| Cyp2c50 cytochrome P450 2C50 isoform 1 |
| Cyp2c54 Cytochrome P450 2C54 |
| Cyp2c55 Cytochrome P450 2C55 |
| Cyp2c68 cytochrome P450, family 2, subfamily c, polypeptide 68 |
| Cyp2d12 Cytochrome P450 2D12 |
| Cyp2d9 cytochrome P450 2D9 |
| Cyp2g1 Olfactory-specific steroid hydroxylase |
| Cyp3a11 Cytochrome P450 3A11 |
| Cyp3a25 Cytochrome P450 3A25 |
| Cyp3a59 Uncharacterized protein |
| Ephx1 Microsomal epoxide hydrolase |
| Fmo5 Dimethylaniline monooxygenase [N-oxide-forming] 5 |
| |

| **Phase 2 drug metabolism** |
|---|
| Gsta1 Glutathione S-transferase A1 |
| Gsta2 Glutathione S-transferase A2 |
| Gsta4 Glutathione S-transferase A4 |
| Gstm3 Glutathione S-transferase Mu 3 |
| Gstt1 glutathione S-transferase theta-1 |
| Gstt2 Glutathione S-transferase theta-2 |
| Sult1d1 sulfotransferase family 1D, member 1 |
| Sult1d1 Tyrosine-ester sulfotransferase |
| Sult2a1 Bile salt sulfotransferase 1 |
| Sult2a5 sulfotransferase family 2A member 1 family member |
| Ugt1a9 UDP-glucuronosyltransferase 1-9 |
| Gstp1 Glutathione S-transferase P 1 |
| |

| **Phase 3** |
|---|
| Abcb11 bile salt export pump |
| Abcb1b Multidrug resistance protein 1 |
| Abcc2 canalicular multispecific organic anion transporter 1 |
| Abcc3 Isoform 1 of Canalicular multispecific organic anion transporter 2 |
| Slc10a1 Sodium/bile acid cotransporter |
| Slc16a1 Monocarboxylate transporter 1 |
| Slc22a18 solute carrier family 22 member 18 |
| Slc2a2 Solute carrier family 2, facilitated glucose transporter member 2 |
| Slc4a1 Isoform Kidney of Band 3 anion transport protein |
| Slco1b2 Isoform 1 of Solute carrier organic anion transporter family member 1 B2 |
| Slco2b1 Solute carrier organic anion transporter family member 2B1 |

As a general conclusion, cocktail treatments triggered much higher expression of constitutively lowly express proteins compared to single compound alone. For example, it has been observed that the combination of TCDD (2,3,7,8-Tetrachlorodibenzodioxin) and TCPOBOP enhanced protein expression in almost all proteins examined except for Cyp2e. Similarly, with the exception of Homx1 and Nqo1, the combined BNF and PB also increased expression of all protein examined. By adding another inducer-PCN to the BNF/PB combination, the expression of Homx1, Nqo1, Cyp3a was greatly improved. The results suggest that the combination of CAR and PXR drug ligands can be highly useful in the induction of drug metabolism enzymes regulated by both nuclear receptors than a single ligand.

### Example 2 - "Drug metabolism" pathway enhanced SILAM improves the quantification of Cyp2B10 in mouse liver compared to the traditional SILAM.

This experiment was conducted to test the utility of the drug metabolism-enhanced reference in an actual SILAM protocol in comparison to normal SILAM protocol. To do this we compared the drug metabolism protein expression in liver from mice treated with TCPOBOP or Ethoxyquin with normal mice, i.e. where a modulatory composition had not been administered.

Liver protein extracts from wild type mice treated with TCPOBOP (1 mg/kg) or Ethoxyquin (1 mg/kg) or corn oil (control) were spiked with liver heavy protein extract from normal SILAM mice or drug metabolism pathway-enhanced SILAM mice according to the present invention.

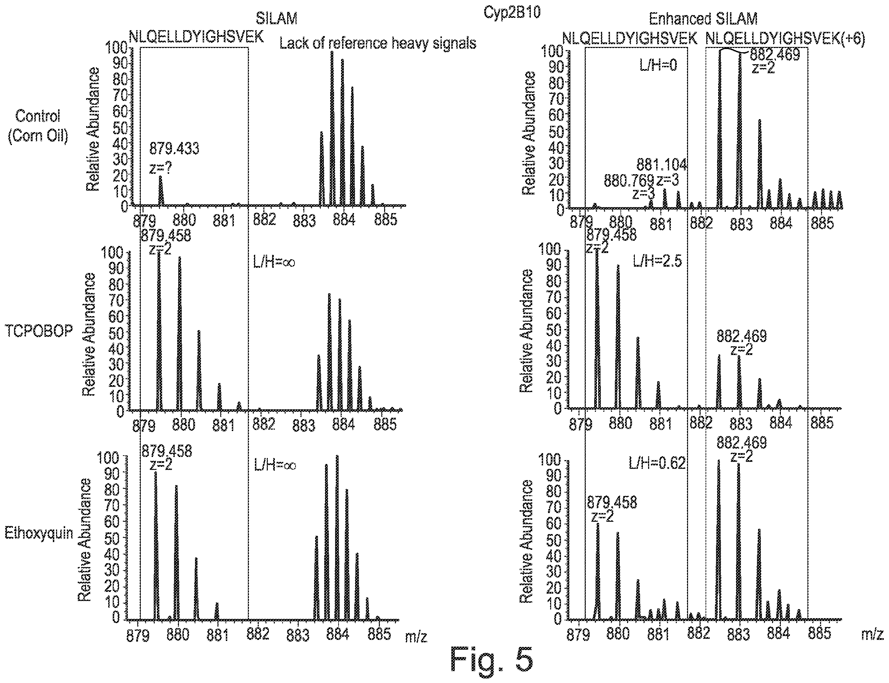

Data from one of peptides of Cyp2B10 (NLQELLDYIGHSVEK - SEQ ID NO 1) are shown in Fig 5.

Note that no detectable heavy peptide signals (in red) were observed in the normal SILAM spectra (thus the calculated Light/Heavy (H/L) ratio for each sample is infinity) - these results are shown in the three spectra on the left hand side of Fig 4. However, in "pathway-enhanced" SILAM, shown in the three right hand spectra, the heavy corresponding peptide signals were clear such that the differential expression ration can then be accurately compared between different inducers.

Using normal SILAM techniques, the measurement of proteins listed in Table 3 is not feasible. This is because Cyp2B10 is not normally expressed at a detectable level in untreated mice. Therefore, when using traditional SILAM, no heavy peptide signal for NLQELLDYIGHSVEK(+6) (SEQ ID NO 1) was detected (Fig 4). In TCPOBOP or Ethoxquin treated mice, this peptide becomes detectable as indicated by the 2+ ion at 879.458. However, since there is no heavy peptide signal to reference to in normal SILAM, the L/H ratios for both TCPOBOP and Ethoxyquin are infinite (and not comparable). In contrast, using drug metabolism pathway-enhanced SILAM, the quantification becomes feasible and reliable with the clear heavy isotope signals at 882.469. The results show that there is no or little expression of Cyp2B10 (L/H=0) in control (corn oil treated), whereas the L/H ratios are 2.50 and 0.62 in TCPOBOP and Ethoxyquin treated mice, respectively. The results suggest that TCPOBOP induces 4 fold higher Cyp2B10 expression than Ethoxyquin.

Treatments using a mixture (cocktail) of different inducers, induced much higher expression of constitutively lowly express proteins compared to single compound alone. This suggests that modulatory composition comprising mixtures of inducers are a very attractive option to promote up-regulation of proteins of interest.

All mice in the abovementioned work were maintained under standard animal house conditions, with free access to food and water, and a 12-h light/12-h dark cycle. All animal work was carried out on male 8-week-old C57BL/6J mice in accordance with the Animal Scientific Procedures Act (1986) and after local ethical review.

### Example 3 - Modulatory composition adapted for inflammation pathway

Male C57BL/6 mice were given a single intraperitoneal injection of lipopolysaccharide (LPS; 100 µg) or vehicle and tissues were harvested 24 hours after dosing. Lung tissues were lysed in 2% SDS buffer by ultrasonic probe and proteins extracted were resolved onto a SDS-PAGE, followed by a filter assisted trypsin digestion procedure. The protein digests were analysed by LC-MS/MS and the data were analysed using Peaks software for database search and Progenesis for quantification. The proteins showing more than 5 times induction are listed in Table 4. The results suggest there are more than 100 inflammation related proteins in the lung can be quantified using the enhanced SILAM.

**Table 4 - list of proteins induced in the lung by LPS treatment in an inflammation pathway enhanced SILAM.**

| |
|---|
| AGT Angiotensinogen |
| ANXA1 Annexin A1 |
| ARHGDIB Rho GDP-dissociation inhibitor 2 |
| ARPC1A;ARPC1B Actin-related protein 2/3 complex subunit 1B |
| AZU1 Azurocidin |
| BPI Bactericidal permeability-increasing protein |
| C3 Complement C3 (Fragment) |
| C4B;C4A complement component 4B preproprotein |
| CALM2;CALM3;CALM1 CALM3 protein |
| CAMP Cathelicidin antimicrobial peptide precursor |
| CANX cDNA FLJ55574, highly similar to Calnexin |
| Carboxypeptidase A2 |
| CAT Catalase |
| CD44 Isoform 1 of CD44 antigen |
| CEACAM1 Isoform B of Carcinoembryonic antigen-related cell adhesion molecule 1 |
| chemokine (C-X-C motif) ligand 1 |
| CH13L1 Cartilage glycoprotein-39 |
| CHIT1 50 kDa protein |
| CHIT1 Isoform 1 of Chitotriosidase-1 |
| Class I histocompatibility antigen AA alpha chain |
| CPSF3L Cleavage and polyadenylation specific factor 3-like |
| CST3 Cystatin-C |
| CTSG Cathepsin G |
| DEFA1;DEFA1B Neutrophil defensin 1 |
| DMBT1 Isoform 4 of Deleted in malignant brain tumors 1 protein |
| DST Isoform 3 of Bullous pemphigoid antigen 1 |
| EEF1A1 Elongation factor 1-alpha 1 |
| ELANE Neutrophil elastase |
| EPS8L1 Isoform 1 of Epidermal growth factor receptor kinase substrate 8-like protein 1 |
| EPX Eosinophil peroxidase |
| EVPL Envoplakin |
| FGG Isoform Gamma-B of Fibrinogen gamma chain |
| G6PD Isoform Long of Glucose-6-phosphate 1-dehydrogenase |
| Galectin-1 |
| GC vitamin D-binding protein precursor |
| GDI1 Rab GDP dissociation inhibitor alpha |
| GDI2 GDP dissociation inhibitor 2 |
| GPI Glucose-6-phosphate isomerase |
| GSN Isoform 2 of Gelsolin |
| HBB Hemoglobin subunit beta |
| Hepatoma-derived protein |
| HMGB2 High mobility group protein B2 |
| HP Haptoglobin |
| HPR Isoform 1 of Haptoglobin-related protein |
| Inslin like growth factor 1 |
| Inslin like growth factor 2 |
| IL-6 |
| ITGAM Integrin alpha-M |
| LACRT Extracellular glycoprotein lacritin |
| LAMP2 Isoform LAMP-2A of Lysosome-associated membrane glycoprotein 2 |
| LCN2 Isoform 1 of Neutrophil gelatinase-associated lipocalin |
| LCP1 Plastin-2 |
| LDHA Isoform 1 of L-lactate dehydrogenase A chain |
| LMNB1 Lamin-B1 |
| LOC100290309 hypothetical protein XP_002348012 |
| LTA4H Isoform 1 of Leukotriene A-4 hydrolase |
| LTF Lactoferrin |
| lymphocyte specific 1 (CRA_a) |
| Metalloproteinase inhibitor 2 |
| MIF Macrophage migration inhibitory factor |
| Mimecan, Osteoglycin |
| MMP9 Matrix metalloproteinase-9 |
| MPO Isoform H7 of Myeloperoxidase |
| MRPS26 28S ribosomal protein S26, mitochondrial |
| MUC16 1518 kDa protein |
| Neutrophil antibiotic peptide NP-4 |
| NID1 Isoform 1 of Nidogen-1 |
| NME1-NME2;NME1;NME2 Isoform 1 of Nucleoside diphosphate kinase B |
| Nucleobindin-1 |
| pancratic α-amylase |
| peptidyl-prolyl isomerase A |
| PFN1 Profilin-1 |
| PGD 6-phosphogluconate dehydrogenase, decarboxylating |
| PLS1 Plastin-1 |
| PLS3 Plastin-3 |
| PPIA Peptidyl-prolyl cis-trans isomerase A |
| Protein C |
| Protein NOV homologue nephrolastoma overexpressed gene |
| Protein S100-A8/A9 |
| PRPH Isoform 1 of Peripherin |
| PRTN3 Myeloblastin |
| PUSL1 Isoform 1 of tRNA pseudouridine synthase-like 1 |
| PYGL Glycogen phosphorylase, liver form |
| QSOX1 Isoform 1 of Sulfhydryl oxidase 1 |
| RBL2 Retinoblastoma-like protein 2 TNF-α |
| RILP Isoform 1 of Rab-interacting lysosomal protein |
| RNASE3 Eosinophil cationic protein |
| ROBO1 Isoform 1 of Roundabout homolog 1 |
| S100A12 Protein S100-A12 |
| S100A6 Protein S100-A6 |
| S100A9 Putative uncharacterized protein S100A9 |
| S100P Protein S100-P |
| SARNP 18 kDa protein |
| SERPINA1 Isoform 1 of Alpha-1-antitrypsin |
| TAGLN2 Transgelin-2 |
| TF 7 kDa protein |
| TKT 37 kDa protein |
| TLN1 Talin-1 |
| TPM1 37 kDa protein |
| TPM3 tropomyosin alpha-3 chain isoform 1 |
| TRAP1 Heat shock protein 75 kDa, mitochondrial |

### Conclusions

The "drug metabolism" pathway-enhanced SILAM methodology described above could be highly useful for preclinical study for new drug candidates. Traditionally, this is performed by antibody based assay such as Western blot, which in many cases suffers from specificity issues due to high sequence homology across protein superfamilies (e.g. CYP). The enhanced SILAM design can overcome this issue, while significantly increasing both the number of proteins measured (for many, antibodies are unavailable) and the confidence with which each is identified, all with increased throughput and at a reduced cost.

The examples described above relate to treating mammals to provide improved reference samples for SILAM techniques. However, the present invention fully comprehends applying corresponding techniques to other animals, plants and microbes.

This method can be further applied in cell culture models, particularly human cells in which can be used as reference internal standards for measurement of constitutively lowly expressed proteins in clinical samples. For example, stem cells or iPS can be cultured in stable isotope medium and differentiated to liver cells. A cocktail of drugs can then be applied to stimulate the expression of human drug metabolism enzymes that are lowly expressed in an unstimulated state. The stimulated cells can then be used to quantify the levels of drug metabolism enzymes in liver biopsy samples from patients, or in liver microsome samples which are routinely used to characterize and predict drug metabolism, particularly drug-drug interaction, for candidate drugs. The ability to quantify >100 drug metabolism enzymes reliably in a singe analysis will be of advantage to the current practice using Western blotting on few key Cyp proteins.

### References

1. Ong, S. E.; Blagoev, B.; Kratchmarova, I.; Kristensen, D. B.; Steen, H.; Pandey, A.; Mann, M., Stable isotope labeling by amino acids in cell culture, SILAC, as a simple and accurate approach to expression proteomics. Mol Cell Proteomics 2002, 1 (5), 376-86.
2. Gruhler, A.; Schulze, W. X.; Matthiesen, R.; Mann, M.; Jensen, O. N., Stable isotope labeling of Arabidopsis thaliana cells and quantitative proteomics by mass spectrometry. Mol Cell Proteomics 2005, 4 (11), 1697-709.
3. Selbach, M.; Mann, M., Protein interaction screening by quantitative immunoprecipitation combined with knockdown (QUICK). Nat Methods 2006, 3 (12), 981-3.
4. McClatchy, D. B.; Liao, L.; Park, S. K.; Venable, J. D.; Yates, J. R., Quantification of the synaptosomal proteome of the rat cerebellum during post-natal development. Genome Res 2007, 17 (9), 1378-88.
5. Vermeulen, M.; Mulder, K. W.; Denissov, S.; Pijnappel, W. W.; van Schaik, F. M.; Varier, R. A.; Baltissen, M. P.; Stunnenberg, H. G.; Mann, M.; Timmers, H. T., Selective anchoring of TFIID to nucleosomes by trimethylation of histone H3 lysine 4. Cell 2007, 131 (1), 58-69.
6. Hanke, S.; Besir, H.; Oesterhelt, D.; Mann, M., Absolute SILAC for accurate quantitation of proteins in complex mixtures down to the attomole level. J Proteome Res 2008, 7 (3), 1118-30.
7. Kruger, M.; Moser, M.; Ussar, S.; Thievessen, I.; Luber, C. A.; Forner, F.; Schmidt, S.; Zanivan, S.; Fassler, R.; Mann, M., SILAC mouse for quantitative proteomics uncovers kindlin-3 as an essential factor for red blood cell function. Cell 2008, 134 (2), 353-64.
8. Liao, L.; McClatchy, D. B.; Park, S. K.; Xu, T.; Lu, B.; Yates, J. R., 3rd, Quantitative analysis of brain nuclear phosphoproteins identifies developmentally regulated phosphorylation events. J Proteome Res 2008, 7 (11), 4743-55.
9. McClatchy, D. B.; Yates, J. R., 3rd, Stable Isotope Labeling of Mammals (SILAM). CSH Protoc 2008, 2008, pdb prot4940.
10. Pan, C.; Gnad, F.; Olsen, J. V.; Mann, M., Quantitative phosphoproteome analysis of a mouse liver cell line reveals specificity of phosphatase inhibitors. Proteomics 2008, 8 (21), 4534-46.
11. Pan, C.; Kumar, C.; Bohl, S.; Klingmueller, U.; Mann, M., Comparative proteomic phenotyping of cell lines and primary cells to assess preservation of cell type-specific functions. Mol Cell Proteomics 2009, 8 (3), 443-50.
12. McClatchy, D. B.; Liao, L.; Park, S. K.; Xu, T.; Lu, B.; Yates lii, J. R., Differential proteomic analysis of mammalian tissues using SILAM. PLoS One 2011, 6(1), e16039.
13. Monetti, M.; Nagaraj, N.; Sharma, K.; Mann, M., Large-scale phosphosite quantification in tissues by a spike-in SILAC method. Nat Methods 2011, 8 (8), 655-8.
14. Zanivan, S.; Krueger, M.; Mann, M., In vivo quantitative proteomics: the SILAC mouse. Methods Mol Biol 2011, 757, 435-50.
15. McClatchy, D. B.; Liao, L.; Lee, J. H.; Park, S. K.; Yates, J. R., 3rd, Dynamics of subcellular proteomes during brain development. J Proteome Res 2012, 11 (4), 2467-79.
16. Craft, G. E.; Chen, A.; Nairn, A. C., Recent advances in quantitative neuroproteomics. Methods 2013**.**
17. Kappei, D.; Butter, F.; Benda, C.; Scheibe, M.; Draskovic, I.; Stevense, M.; Novo, C. L.; Basquin, C.; Araki, M.; Araki, K.; Krastev, D. B.; Kittler, R.; Jessberger, R.; Londono-Vallejo, J. A.; Mann, M.; Buchholz, F., HOT1 is a mammalian direct telomere repeat-binding protein contributing to telomerase recruitment. EMBO J 2013, 32 (12), 1681-701.
18. Rauniyar, N.; McClatchy, D. B.; Yates, J. R., 3rd, Stable isotope labeling of mammals (SILAM) for in vivo quantitative proteomic analysis. Methods 2013**.**
19. Rayavarapu, S.; Coley, W.; Cakir, E.; Jahnke, V.; Takeda, S.; Aoki, Y.; Grodish-Dressman, H.; Jaiswal, J. K.; Hoffman, E. P.; Brown, K. J.; Hathout, Y.; Nagaraju, K., Identification of Disease Specific Pathways Using in Vivo SILAC Proteomics in Dystrophin Deficient mdx Mouse. Mol Cell Proteomics 2013, 12 (5), 1061-73.
20. Zanivan, S.; Meves, A.; Behrendt, K.; Schoof, E. M.; Neilson, L. J.; Cox, J.; Tang, H. R.; Kalna, G.; van Ree, J. H.; van Deursen, J. M.; Trempus, C. S.; Machesky, L. M.; Linding, R.; Wickstrom, S. A.; Fassler, R.; Mann, M., In vivo SILAC-based proteomics reveals phosphoproteome changes during mouse skin carcinogenesis. Cell Rep 2013, 3 (2), 552-66.

## Claims

1. A method for preparing a labelled reference sample of biological material for use in mass spectrometry-based protein detection or analysis, the method comprising:
a) providing a protein expression system;
b) providing said protein expression system with a nutrient composition comprising a source of amino acids or components for the synthesis of amino acids, wherein at least a portion of said amino acids or components for the synthesis of amino acids are labelled with a non-radioactive or radioactive isotope;
c) administering to said protein expression system a modulatory composition, wherein said modulatory composition increases abundance of one or more proteins in the protein expression system;
d) allowing said protein expression system to incorporate components of the nutrient composition into proteins during translation.

2. The method of claim 1 wherein said protein expression system is a biological cell, biological tissue, an organ, a non-human organism, a collection of non-human organisms, a portion of an organism, and a cell-free biological mimetic system.

3. The method of claim 1 or 2 wherein said protein expression system is a non-human animal, more preferably a non-human mammal, yet more preferably a mouse or rat.

4. The method of any preceding claim wherein said protein expression system is a non-human organism, and which comprises maintaining provision of said nutrient composition for sufficient time for said organism to generate at least one generation of offspring, and then providing at least one of said offspring organisms with said nutrient composition,
and preferably
maintaining provision of said nutrient composition to said at least one offspring organisms, for sufficient time for said organism to generate at another generation of offspring (F2 generation), and selecting one or more F2 offspring of the parent organism to provide a reference sample of biological material.

5. The method of any preceding claim wherein the modulatory composition comprises one or more of TCPOBOP, rafamycin, pregnenolone 16α-carbonitrile (PCN), ethoxyquin, and phenobarbital.

6. The method of any preceding claim wherein the modulatory composition is administered to induce expression of proteins involved in drug metabolism (e.g. P450 isoforms), inflammation or oxidative stress.

7. The method of any preceding claim wherein said nutrient composition comprises amino acids or amino acid precursors which are enriched for at least one isotope chosen from the group consisting of nitrogen-15, carbon-13, oxygen-17, oxygen-18, sulphur-34, selenium-74, selenium-76, selenium-77, selenium-78, selenium-82 and hydrogen-2.

8. A method for determining the relative abundance of one or more proteins of interest in samples of biological matter, the method comprising:
a) providing a first protein expression system;
b) providing a second protein expression system;
c) applying a test condition to at least one of said first or second protein expression systems;
d) providing an isotope-labelled reference sample comprising an equivalent protein expression system to those in a) and b), wherein said reference sample is obtained or obtainable by the method of any one of claims 1 to 7;
e) obtaining a first sample from the first protein expression system;
f) obtaining a second sample from the second protein expression system;
g) combining at least a portion of the first sample with a portion of the reference sample to form a first combined sample;
h) combining at least a portion of the second sample with a portion of the reference sample to form a second combined sample;
i) isolating or enriching one or more proteins from each of said first and second combined samples;
j) subjecting the isolated or enriched proteins protein to mass spectroscopy to develop a mass spectrum;
k) computing a ratio between the peak intensities of at least one pair of closely spaced peaks;
l) determining the relative abundance of a protein in each sample compared to the reference sample based on the at least one computed ratio; and optionally
m) identifying the protein.

9. The method of claim 8, further comprising digesting the at least one protein of interest, suitably wherein the at least one protein of interest is digested by trypsin.

10. The method of claim 8 or 9 comprising:
- removing a plurality of proteins of interest from the combined samples;
- digesting the plurality of proteins of interest into a plurality of peptides;
- subjecting the digested proteins to mass spectroscopy to develop the mass spectrum;
- selecting a plurality of pairs of closely spaced peaks on the mass spectrum;
- computing the ratio of the intensities of the peaks in each pair;
- determining the protein from which the pair of peaks in the mass spectrum are derived based on the mass spectrum; and
- determining the relative abundance of the protein in each sample.

11. The method of any one of claims 8 to 10 comprising determining the relative quantity of a modified protein of interest in each sample, suitably wherein the type of peptide modification is chosen from the group consisting of phosphorylation, glycosylation, methylation, S-glutathionylation, ubiquitination, SUMOylation, pupylation, oxidation, succinylation, sulfation and acylation of the peptide, the determining step comprising determining the difference in the relative abundance of the modified peptide in each sample, and optionally
determining the site of the modification on the protein.

12. The method of any one of claims 8 to 11, wherein step c) comprises subjecting one of the said first and second protein expression systems to an environmental or chemical stimulus; or
wherein the first and second protein expression systems are non-human organisms and step c) comprises genetically manipulating one of the said first and second non-human organisms; or
wherein step c) comprises subjecting the one of the said first and second protein expression systems to a treatment chosen from the group consisting of a virus, a bacteria and a carcinogen.

13. The method of any one of claims 8 to 12 wherein the first and second protein expression systems are identical,
and preferably wherein the protein expression system of the reference sample is identical to the first and second protein expression systems.

## Patentansprüche

1. Verfahren zur Zubereitung einer markierten Referenzprobe einer biologischen Substanz zur Verwendung beim Proteinnachweis bzw. der Proteinanalyse auf der Basis von Massenspektrometrie, wobei das Verfahren folgendes umfasst:
a) Bereitstellen eines Proteinexpressionssystems;
b) Bereitstellen des Proteinexpressionssystems mit einer Nährstoffzusammensetzung, umfassend eine Quelle von Aminosäuren oder Komponenten für die Synthese von Aminosäuren, wobei wenigstens ein Teil der Aminosäuren oder der Komponenten für die Synthese von Aminosäuren mit einem nichtradioaktiven oder radioaktiven Isotop markiert ist;
c) Verabreichen einer regulierenden Zusammensetzung zu dem Proteinexpressionssystem, wobei die regulierende Zusammensetzung die Abundanz eines oder mehrerer Proteine in dem Proteinexpressionssystem erhöht;
d) Ermöglichen, dass das Proteinexpressionssystem Komponenten der Nährstoffzusammensetzung während der Translation in Proteine inkorporiert.

2. Verfahren nach Anspruch 1, wobei das Proteinexpressionssystem eine biologische Zelle, biologisches Gewebe, ein Organ, ein nichthumaner Organismus, eine Sammlung nichthumaner Organismen, ein Teil eines Organismus und ein zellfreies biologisches mimetisches System ist.

3. Verfahren nach Anspruch 1 oder 2, wobei das Proteinexpressionssystem ein nichthumanes Tier ist, bevorzugter ein nichthumanes Säugetier, darüber hinaus bevorzugt eine Maus oder eine Ratte.

4. Verfahren nach einem der vorstehenden Ansprüche, wobei das Proteinexpressionssystem ein nichthumaner Organismus ist, und wobei das Verfahren das Aufrechterhalten der Bereitstellung der Nährstoffzusammensetzung über einen ausreichenden Zeitraum umfasst, damit der Organismus wenigstens eine Generation von Abkömmlingen erzeugen kann, und wobei danach wenigstens einer der Abkömmlingsorganismen mit der Nährstoffzusammensetzung versorgt wird;
und vorzugsweise
Aufrechterhalten der Bereitstellung der Nährstoffzusammensetzung an den wenigstens einen Abkömmlingsorganismus über einen ausreichenden Zeitraum, damit der Organismus eine zweite Generation von Abkömmlingen (F2 Generation) erzeugen kann, und Auswählen eines oder mehrerer F2 Abkömmlinge des Mutterorganismus zur Bereitstellung einer Referenzprobe einer biologischen Substanz.

5. Verfahren nach einem der vorstehenden Ansprüche, wobei die regulierende Zusammensetzung eines oder mehrere der folgenden umfasst: TCPOBOP, Rafamycin, Pregnenolon, 16α-Carbonitril (PCN), Ethoxyquin und Phenobarbital.

6. Verfahren nach einem der vorstehenden Ansprüche, wobei die regulierende Zusammensetzung verabreicht wird, um die Expression von Proteinen zu induzieren, die beteiligt sind am Arzneimittelstoffwechsel (z.. P450 Isoformen), Entzündung und oxidativem Stress.

7. Verfahren nach einem der vorstehenden Ansprüche, wobei die Nährstoffzusammensetzung Aminosäuren oder Aminosäurenvorläufer umfasst, die angereichert sind für wenigstens ein Isotop, das ausgewählt ist aus der Gruppe bestehend aus Stickstoff-15, Kohlenstoff-13, Sauerstoff-17, Sauerstoff-18, Schwefel-34, Selenium-74, Selenium-76, Selenium-77, Selenium-78, Selenium-82 und Wasserstoff-2.

8. Verfahren zur Bestimmung der relativen Abundanz eines oder mehrerer relevanter Proteine in Proben von biologischem Material, wobei das Verfahren folgendes umfasst:
a) Bereitstellen eines ersten Proteinexpressionssystems;
b) Bereitstellen eines zweiten Proteinexpressionssystems;
c) Anwenden einer Bestbedingung auf wenigstens eines des ersten oder zweiten Proteinexpressionssystems;
d) Bereitstellen einer Isotop-markierten Referenzprobe, umfassend ein äquivalentes Proteinexpressionssystem zu denen unter a) und b), wobei die Referenzprobe erhalten wird oder erhalten werden kann durch das Verfahren nach einem der Ansprüche 1 bis 7;
e) Erhalten einer ersten Probe aus dem ersten Proteinexpressionssystem;
f) Erhalten einer zweiten Probe aus dem zweiten Proteinexpressionssystem;
g) Kombinieren wenigstens eines Teils der ersten Probe mit einem Teil der Referenzprobe, um eine erste kombinierte Probe zu bilden;
h) Kombinieren wenigstens eines Teils der zweiten Probe mit einem Teil der Referenzprobe, um eine zweite kombinierte Probe zu bilden;
i) Isolieren oder Anreichern eines oder mehrerer Proteine aus jeder der ersten und zweiten kombinierten Proben;
j) Unterziehen der isolierten oder angereicherten Proteine einer Massenspektrometrie, um ein Massenspektrum zu entwickeln;
k) Berechnen eines Verhältnisses zwischen Spitzenintensitäten wenigstens eines Paars dicht beabstandeter Spitzen;
l) Bestimmen der relativen Abundanz eines Proteins in jeder Probe im Vergleich zu der Referenzprobe auf der Basis des wenigstens einen berechneten Verhältnisses; und optional
m) Identifizieren des Proteins.

9. Verfahren nach Anspruch 8, ferner umfassend das Verdauen des wenigstens einen relevanten Proteins, wobei das wenigstens eine relevante Protein in geeigneter Weise durch Trypsin verdaut wird.

10. Verfahren nach Anspruch 8 oder 9, umfassend:
- Entfernen mehrerer relevanter Proteine aus den kombinierten Proben;
- Verdauen der mehreren relevanten Proteine in mehrere Peptide;
- Unterziehen der verdauten Proteine einer Massenspektrometrie, um das Massenspektrum zu entwickeln;
- Auswählen mehrerer Paare dicht beabstandeter Spitzen in dem Massenspektrum;
- Berechnen des Verhältnisses der Intensitäten der Spitzen in jedem Paar;
- Bestimmen des Proteins, aus dem die Paare der Spitzen in dem Massenspektrum auf der Basis des Massenspektrums abgeleitet werden; und
- Bestimmen der relativen Abundanz des Proteins in jeder Probe.

11. Verfahren nach einem der Ansprüche 8 bis 10, umfassend das Bestimmen der relativen Menge eines modifizierten relevanten Proteins in jeder Probe, wobei der Typ der Peptidmodifikation in geeigneter Weise ausgewählt wird aus der Gruppe bestehend aus Phosphorylierung, Glykosylierung, Methylierung, S-Glutathionylierung, Ubiquitinierung, Sumoylierung, Pupylierung, Oxidation, Succinylierung, Sulfatierung und Acylierung des Peptids, wobei der Schritt des Bestimmens das Bestimmen der Differenz der relativen Abundanz des modifizierten Peptids in jeder Probe umfasst, und optional
das Bestimmen der Stelle der Modifikation an dem Protein.

12. Verfahren nach einem der Ansprüche 8 bis 11, wobei Schritt c) das Aussetzen des ersten oder des zweiten Proteinexpressionssystems einem Reiz aus der Umgebung oder einem chemischen Reiz umfasst; oder
wobei das erste und das zweite Proteinexpressionssystem nichthumane Organismen sind; und
wobei Schritt c) das genetische Manipulieren des ersten oder des zweiten nichthumanen Organismus umfasst; oder
wobei Schritt c) das Aussetzen des ersten oder des zweiten Proteinexpressionssystems einer Behandlung umfasst, die ausgewählt ist aus der Gruppe bestehend aus einem Virus, einem Bakterium und einem Karzinogen.

13. Verfahren nach einem der Ansprüche 8 bis 12, wobei das erste und das zweite Proteinexpressionssystem identisch sind;
und wobei vorzugsweise das Proteinexpressionssystem der Referenzprobe identisch ist mit dem ersten und dem zweiten Proteinexpressionssystem.

## Revendications

1. Procédé de préparation d'un échantillon de référence marqué de matériel biologique destiné à être utilisé dans une détection ou analyse protéique par spectrométrie de masse, le procédé comprenant les étapes consistant à :
a) fournir un système d'expression protéique ;
b) fournir ledit système d'expression protéique avec une composition nutritive comprenant une source d'acides aminés ou de composants pour la synthèse d'acides aminés, au moins une partie desdits acides aminés ou composants pour la synthèse d'acides aminés étant marqués avec un isotope radioactif ou non radioactif ;
c) administrer audit système d'expression protéique une composition modulatrice, ladite composition modulatrice augmentant l'abondance d'une ou de plusieurs protéines dans le système d'expression protéique ;
d) permettre audit système d'expression protéique d'incorporer des composants de la composition nutritive dans les protéines pendant la traduction.

2. Procédé selon la revendication 1, ledit système d'expression protéique étant une cellule biologique, un tissu biologique, un organe, un organisme non humain, un ensemble d'organismes non humains, une partie d'un organisme et un système mimétique biologique sans cellule.

3. Procédé selon la revendication 1 ou 2, ledit système d'expression protéique étant un animal non humain, de préférence un mammifère non humain, et de préférence encore une souris ou un rat.

4. Procédé selon l'une quelconque des revendications précédentes, ledit système d'expression protéique étant un organisme non humain, comprenant les étapes consistant à maintenir la fourniture de ladite composition nutritive pendant suffisamment de temps pour que ledit organisme produise au moins une génération de descendance, puis à fournir au moins un desdits organismes descendants avec ladite composition nutritive, et de préférence
à maintenir la fourniture de ladite composition nutritive audit au moins un organisme descendant, pendant suffisamment de temps pour que ledit organisme génère une autre génération de descendants (génération F2), et à sélectionner un ou plusieurs descendants F2 de l'organisme parent pour fournir un échantillon de référence de matériel biologique.

5. Procédé selon l'une quelconque des revendications précédentes, la composition modulatrice comprenant TCPOBOP, rafamycine, prégnénolone 16α-carbonitrile (PCN), éthoxyquine, et phénobarbital.

6. Procédé selon l'une quelconque des revendications précédentes, la composition modulatrice étant administrée pour induire l'expression de protéines impliquées dans le métabolisme de médicament (p. ex. isoformes P450), l'inflammation ou le stress oxydatif.

7. Procédé selon l'une quelconque des revendications précédentes, ladite composition nutritive comprenant des acides aminés ou des précurseurs d'acide aminé qui sont enrichis pour au moins un isotope choisi dans le groupe consistant en azote-15, carbone-13, oxygène-17, oxygène-18, soufre-34, sélénium-74, sélénium-76, sélénium-77, sélénium-78, sélénium-82 et hydrogène-2.

8. Procédé pour déterminer l'abondance relative d'une ou de plusieurs protéines d'intérêt dans des échantillons de matériel biologique, le procédé comprenant les étapes consistant à :
a) fournir un premier système d'expression protéique ;
b) fournir un second système d'expression protéique ;
c) appliquer une condition d'essai audit premier et/ou second système d'expression protéique ;
d) fournir un échantillon de référence marqué par isotope comprenant un système d'expression protéique équivalant à ceux dans a) et b), ledit échantillon de référence étant obtenu ou pouvant être obtenu par le procédé selon l'une quelconque des revendications 1 à 7 ;
e) obtenir un premier échantillon du premier système d'expression protéique ;
f) obtenir un second échantillon du second système d'expression protéique ;
g) combiner au moins une partie du premier échantillon avec une partie de l'échantillon de référence pour former un premier échantillon combiné ;
h) combiner au moins une partie du second échantillon avec une partie de l'échantillon de référence pour former un second échantillon combiné ;
i) isoler ou enrichir une ou plusieurs protéines à partir de chacun desdits premier et second échantillons combinés ;
j) soumettre la protéine protéique isolée ou enrichie à la spectroscopie de masse pour développer un spectre de masse ;
k) calculer un rapport entre les intensités de crête d'au moins une paire de pics rapprochés ;
l) déterminer l'abondance relative d'une protéine dans chaque échantillon par rapport à l'échantillon de référence en se basant sur l'au moins un rapport calculé ; et éventuellement
m) identifier la protéine.

9. Procédé selon la revendication 8, comprenant en outre l'étape consistant à digérer l'au moins une protéine d'intérêt, de façon appropriée l'au moins une protéine d'intérêt étant digérée par la trypsine.

10. Procédé selon la revendication 8 ou 9, comprenant les étapes consistant à :
- retirer une pluralité de protéines d'intérêt des échantillons combinés ;
- digérer la pluralité de protéines d'intérêt en une pluralité de peptides ;
- soumettre les protéines digérées à la spectroscopie de masse pour développer le spectre de masse ;
- sélectionner une pluralité de paires de pics étroitement espacés sur le spectre de masse ;
- calculer le rapport des intensités des pics dans chaque paire ;
- déterminer la protéine de laquelle la paire de pics dans le spectre de masse est dérivée en fonction du spectre de masse ; et
- déterminer l'abondance relative de la protéine dans chaque échantillon.

11. Procédé selon l'une quelconque des revendications 8 à 10, comprenant l'étape consistant à déterminer la quantité relative d'une protéine modifiée d'intérêt dans chaque échantillon, de façon appropriée le type de modification peptidique étant choisi dans le groupe consistant en phosphorylation, glycosylation, méthylation, S-glutathionylation, ubiquitination, SUMOylation, pupylation, oxydation, succinylation, sulfation et acylation du peptide, la détermination comprenant les étapes consistant à déterminer la différence dans l'abondance relative du peptide modifié dans chaque échantillon, et éventuellement
à déterminer le site de la modification sur la protéine.

12. Procédé selon l'une quelconque des revendications 8 à 11, l'étape c) comprenant l'étape consistant à soumettre l'un desdits premier et second systèmes d'expression protéique à un stimulus environnemental ou chimique ; ou
les premier et second systèmes d'expression protéique étant des organismes non humains et l'étape c) comprenant l'étape consistant à manipuler génétiquement l'un desdits premier et second organismes non humains ; ou
l'étape c) comprenant l'étape consistant à soumettre l'un desdits premier et second systèmes d'expression protéique à un traitement choisi dans le groupe consistant en un virus, une bactérie et un carcinogène.

13. Procédé selon l'une quelconque des revendications 8 à 12, les premier et second systèmes d'expression protéique étant identiques,
et de préférence le système d'expression protéique de l'échantillon de référence étant identique aux premier et second systèmes d'expression protéique.
